# EUROPEAN PATENT SPECIFICATION

(11) **EP 3 302 458 B1**
(45) Date of publication and mention of the grant of the patent: **07.09.2022**
(21) Application number: 16800769.8
(22) Date of filing: 27.05.2016
(51) Int. Cl.: A61K 31/198, A61K 31/195, A61K 9/48, A61K 9/52, A61K 45/06, A61K 47/24, A61K 9/127, A61P 37/00, A61P 3/10

(54) **METHODS OF PREVENTING AND TREATING AUTOIMMUNITY**
VERFAHREN ZUR PRÄVENTION UND BEHANDLUNG VON AUTOIMMUNITÄT
MÉTHODES DE PRÉVENTION ET DE TRAITEMENT DE L'AUTO-IMMUNITÉ

(30) Priority: 28.05.2015 US 201562167844 P
(43) Date of publication of application: 11.04.2018
(73) Proprietor: The Regents of The University of Colorado, A Body Corporate, Denver, CO 80203 (US); The University of Florida Research Foundation Incorporated, Gainesville, FL 32611 (US)
(72) Inventor: ANCHORDOQUY, Thomas J., Lakewood, CO 80215 (US); GOTTLIEB, Peter A., Englewood, CO 80111 (US); MICHELS, Aaron, Aurora, CO 80015 (US); OSTROV, David, Gainesville, FL 32607 (US)
(74) Representative: Marks & Clerk LLP
(86) International application number: PCT/US2016/034527
(87) International publication number: WO 2016/191634

(56) References cited:
- WO-A1-84/02843
- JP-A- H0 952 847
- US-A- 4 783 337
- US-A1- 2004 253 276
- US-A1- 2005 222 270
- US-A1- 2013 017 262
- US-A1- 2014 050 807
- Aaron Michels: "Targeting the Trimolecular Complex for Immune Intervention", ATDC 2014 Keystone Conference, 18 July 2014 (2014-07-18), XP055533330, Retrieved from the Internet: URL:http://www.ucdenver.edu/academics/coll eges/medicalschool/centers/BarbaraDavis/Do cuments/ATDC%202014%20Slides/3.4%20Michels %20Targeting%20Trimolecular%20Complex.pdf [retrieved on 2018-12-12]
- A. W. MICHELS ET AL: "Structure-Based Selection of Small Molecules To Alter Allele-Specific MHC Class II Antigen Presentation", THE JOURNAL OF IMMUNOLOGY, vol. 187, no. 11, 31 October 2011 (2011-10-31), pages 5921-5930, XP055533746, US ISSN: 0022-1767, DOI: 10.4049/jimmunol.1100746
- DAVID A. OSTROV ET AL: "Methyldopa blocks MHC class II binding to disease-specific antigens in autoimmune diabetes", JOURNAL OF CLINICAL INVESTIGATION, vol. 128, no. 5, 3 April 2018 (2018-04-03) , pages 1888-1902, XP055533336, GB ISSN: 0021-9738, DOI: 10.1172/JCI97739

## Description

### TECHNICAL FIELD

The disclosure relates to methyldopa, extended-release pharmaceutical compositions containing the same, and their use in the prevention or treatment of autoimmune diseases, such as autoimmune diabetes.

### BACKGROUND

Autoimmune disorders are diseases caused by the body producing an inappropriate immune response against its own tissues, in which the immune system creates T lymphocytes and autoantibodies that attack one's own cells, tissues, and/or organs. Researchers have identified 80-100 different autoimmune diseases and suspect at least 40 additional diseases have an autoimmune basis.

Autoimmune disorders are classified into two types, organ-specific (directed mainly at one organ) and non-organ-specific (widely spread throughout the body). Examples of organ-specific autoimmune disorders are insulin-dependent Type 1 diabetes, which affects the pancreas, Hashimoto's thyroiditis and Graves' disease, which affects the thyroid gland, pernicious anemia, which affects the stomach, Addison's disease, which affects the adrenal glands, chronic active hepatitis, which affects the liver and myasthenia gravis. which affects the muscles. Examples of non-organ-specific autoimmune disorders are rheumatoid arthritis, multiple sclerosis, and lupus.

One of the most prevalent organ-specific autoimmune diseases, Type 1 diabetes, is characterized by the production of autoantibodies that target the insulin-secreting pancreatic beta cells. The destruction of the beta cells is mainly due to the action of T cells. In most cases, T cells can respond to an antigen only when the antigen is properly presented by an antigen presenting cell expressing the appropriate major histocompatibility complex (MHC) molecule. Thus, T cell immune response to an antigen requires recognition by the T cell receptor of an antigen coupled to a MHC molecule, and this recognition requires the assembly of a tri-molecular complex between an antigen, a MHC molecule and T cell receptor.

Evidence strongly indicates that insulin/proinsulin is a key or primary auto-antigen in the development of type 1 diabetes in the NOD (non-obese diabetic) mouse model. Initial cloning of T cells from islets of NOD mice led to the discovery that the native insulin B chain amino acids 9-23 (B:9-23 insulin peptide) is the dominant antigenic peptide epitope presented by the class II MHC molecule I-A. Mice lacking the native B:9-23 sequence fail to develop diabetes and development of insulin autoantibodies and insulitis are markedly decreased. Restoring the native B:9-23 sequence with an islet transplant (but not bone marrow transplant) or peptide immunization, or a native proinsulin transgene, restores anti-insulin autoimmunity and generates CD4 T cells that cause diabetes.

The major genetic determinant of islet autoimmunity and diabetes in human and animal models are genes within the major histocompatibility complex, and in particular, class II MHC alleles. The NOD mouse's unique sequence of IA (homologous to human DQ) and lack of expression of I-E (shared with many standard mouse strains) are essential for the development of diabetes.

Michels (ATDC 2014 keystone Conference. 18^{th} July 2014) discloses immunological aspects of Type-1 diabetes and the blocking of DQ8 by methyldopa.

There exists a need in the art for safer and more effective methods for treating and preventing autoimmune disorders, such as autoimmune diabetes (type 1 diabetes) and Celiac disease (gluten sensitivity). The instant invention addresses these needs by providing molecules and formulations useful in the treatment and prevention of autoimmune diseases while achieving other advantages discussed more fully below.

### SUMMARY

The present invention provides an extended release formulation of methyldopa for use in delaying the onset or the treatment of an autoimmune disorder including T1D and/or Celiac disease according to any one of claims 1-10, 13 and 14 and the pharmaceutical composition of claims 11, 12 and 14.

The references to methods of treatment in the summary and description of embodiments in this description are to be interpreted as references to the compounds, pharmaceutical compositions and medicaments of the present invention for use in a method for treatment of the human (or animal) body by therapy.

This disclosure provides new uses of methyldopa to prevent or reduce the binding of T cell receptors to peptides presented by class II MHC molecules, as well as therapeutic uses of methyldopa and formulations comprising methyldopa to prevent or slow the formation of autoimmune diabetes (type 1 diabetes; T1D) and Celiac disease in an animal or human.

Many autoimmune disorders have strong associations with specific HLA alleles, including T1D, which is the immune mediated form of diabetes resulting from the chronic autoimmune destruction of pancreatic beta cells. The disease pathogenesis involves T cell infiltration into the islets of the pancreas, which subsequently destroys insulin producing beta cells, and results in overt symptoms of disease. Approximately 90% of all individuals with T1D have DQ8 and/or DQ2 alleles with a predominance of DQ8 (DQA^{∗}0301, DQB^{∗}0302) in 50-60% of all T1D patients. DQ8 and DQ2 alleles confer significant disease risk while another DQ allele, DQ6 (DQB^{∗}0602), provides dominant protection from diabetes development. DQ8 and DQ2 are also the predominant HLA alleles in Celiac disease, present in about 99% of all Celiac disease patients. T1D is now a predictable disease with the measurement of islet autoantibodies (insulin, glutamic acid decarboxylase, insulinoma associated antigen 2, and zinc transporter 8), but it cannot yet be prevented. Furthermore, T1D incidence is increasing 3-5% every year in industrialized countries with children less than five years of age being the most affected.

At the current time, there is no known cure for T1D, and treatment for this disease consists of lifelong administration of insulin. Despite treatment with insulin therapy, long-term complications, including nephropathy, retinopathy, neuropathy, and cardiovascular disease can result. While the progress to complete insulin dependence occurs quickly after clinical onset, initially after diagnosis the pancreas is still able to produce a significant amount of insulin. The Diabetes Control and Complications Trial (DCCT) found that 20% of patients studied, who were within 5 years of diagnosis, had remaining insulin production (0.2-0.5 pmol/ml). Thus, immunologic intervention during this window following diagnosis can potentially save beta cell function and reduce reliance on insulin administration.

Class II major histocompatability molecules are the primary susceptibility locus for many autoimmune diseases, including type 1 diabetes. "Diabetogenic" alleles HLA-DQ8 in humans and I-A^{g7} in non-obese diabetic (NOD) mice confer disease risk, and both molecules share structural similarities. The present inventors have evaluated a novel pathway to identify safe and specific therapies to treat the underlying T cell autoimmunity in T1D. This pathway involves blocking allele-specific MHC class II antigen presentation as a treatment to inhibit DQ8 mediated T cell responses. DQ8 confers significant disease risk by presenting epitopes of insulin and other beta cell antigens to effector CD4 T cells. The present inventors have surprisingly found that methyldopa blocks or reduces insulin and gliadin peptide presentation to T cells. Without intending to be bound by theory, it is believed that methyldopa occupies a pocket along the DQ8 (DQA^{∗}03:01, DQB^{∗}03:02) peptide binding groove, thereby blocking *in vitro* DQ8 restricted T cell responses, and inhibiting DQ8 antigen presentation *in vivo*. Blocking HLA-DQ8 antigen presentation in this way may help preserve beta cell mass (and endogenous insulin production) in new onset T1D and may also prevent T1D onset in multiple islet autoantibody positive individuals (i.e., greater than 2 autoantibodies), 70-90% of whom develop diabetes within 10 years.

This disclosure provides methods of reducing the binding of T cell receptors to insulin/proinsulin peptides presented by class II MHC molecules, to prevent or treat T1D in an individual having or suspected of developing T1D, comprising administering methyldopa to such individuals. This disclosure also provides pharmaceutical compositions containing methyldopa that are particularly useful in such methods of preventing or treating T1D.

Disclosed is a method of inhibiting an autoimmune disease by administering to an individual in need of such treatment, a therapeutically effective amount of methyldopa that inhibits the T cell response to the targeted antigenic peptide of the autoimmune disease. In a preferred aspect of this embodiment, the methyldopa inhibits the binding of a DQ8 peptide to an MHC class II molecule for presentation to CD4+ T cells, thereby preventing the development of autoimmune diabetes or Celiac disease.

Disclosed is a method of preventing, treating or ameliorating autoimmune disorders (including T1D and Celiac disease) by administering to an individual in need of such treatment, a therapeutically effective amount of methyldopa to the individual. The methyldopa is administered in an extended release pharmaceutical formulation as defined in the claims, wherein the methyldopa component of the pharmaceutical formulation is absorbed into the body of the individual over a period of between about 14 hours to about 24 hours. The extended release methyldopa formulation may be administered orally, and the methyldopa component of the pharmaceutical formulation is not completely bioavailable for a period of between about 12 hours to about 24 hours, following oral administration of the formulation to the individual. The extended release pharmaceutical formulations comprise an oil-in-water emulsion. The oil-in-water emulsion comprises an oil phase consisting of olive oil and lecithin. In example embodiments, the oil-in-water emulsion comprises an oil phase comprising olive oil and lecithin, in a ratio of 3:1 w/w.

The disclosure provides an extended release formulation of methyldopa for use in the treatment of autoimmune disorders (including T1D and Celiac disease). This extended release formulation is designed to be released from the formulation to the body of an individual over a period of between about 14 hours to about 24 hours, following oral administration of the formulation to the individual.

Also disclosed is a method of selectively treating T1D in an individual, including selecting an individual for treatment with methyldopa on the basis of the individual having at least two islet autoantibodies detectable in a blood sample from the individual, and selectively administering methyldopa to that individual.

In any of these methods, the individual may be between 1 year and 15 years of age. Alternatively, in these methods, the individual may be between 15 years and 30 years of age. Alternatively, in these methods, the individual may be older than 30 years of age.

In any of these methods, the individual may be administered a dosage of methyldopa between about 50 mg and about 3000 mg of methyldopa per day. Preferably, the individual is administered a dosage of methyldopa between about 250 mg and about 1000 mg of methyldopa per day. The individual may be administered a dosage of methyldopa between about 250 mg and about 500 mg in an oral immediate release dosage formulation at least twice daily (not according to the invention). The individual may be administered a single daily dosage of an extended-release pharmaceutical formulation of methyldopa.

In any of these methods, in addition to methyldopa, the individual may also be administered an anti-diabetic compound selected from at least one of an alpha-glucosidase inhibitor, a biguanide, a Dpp-4 inhibitor, a meglitinide, a sulfonylurea, a thiazolidinedione or combinations thereof.

In any of these methods, the individual may have been tested for the presence of antibodies to a MHC class II molecule bound to an insulin protein or to a peptide fragment of an insulin protein, wherein the presence of antibodies that recognize the MHC class II molecules is indicative of the presence or likely development of T1D. Thus, the present disclosure also provides a method comprising treating an individual found to have antibodies to a MHC class II molecule bound to an insulin protein or to a peptide fragment of an insulin peptide by administering methyldopa to the individual.

Disclosed is a pharmaceutical composition comprising methyldopa formulated in an extended release pharmaceutical formulation, wherein the methyldopa component of the pharmaceutical formulation is released from the formulation to the body of an individual over a period of between about 14 hours to about 24 hours, following oral administration of the formulation to the individual. The extended release pharmaceutical formulation comprises an oil-in-water emulsion.

The oil-in-water emulsion comprises an oil phase consisting of olive oil and lecithin (3:1 w/w). Also disclosed are methods of delaying the onset of autoimmune diabetes in an individual comprising administering to the individual a pharmaceutical composition comprising methyldopa, wherein the methyldopa is administered in an extended release pharmaceutical formulation, wherein the methyldopa component of the pharmaceutical formulation is absorbed by the body of the individual over a period of between about 14 hours to about 24 hours, following oral administration of the formulation to the individual.

Also disclosed are methods of monitoring and adjusting the dosage of methyldopa administered to an individual having or suspected of developing an autoimmune disorder (such as T1D or Celiac disease) including receiving a blood sample from an individual having or suspected of developing the autoimmune disorder that has been administering methyldopa and determining the DQ8-stimulated response of IL-2 T cells in the blood sample. The DQ8-stimulated response of IL-2 T cells in the blood sample are compared to a control level of DQ8-stimulated response of IL-2 T cells in blood samples from at least one of a patient having the autoimmune disorder and a wild type subject known to be free of the disorder. The dosage and/or the frequency of the methyldopa administered to the individual is increased if the DQ8-stimulated response of IL-2 T cells in the blood sample from the individual is statistically similar to the DQ8-stimulated response of IL-2 T cells from the control level in the T1D patient. Alternatively, the dosage and/or the frequency of the methyldopa administered to the individual is maintained or decreased if the DQ8-stimulated response of IL-2 T cells in the blood sample from the individual is statistically similar to the DQ8-stimulated response of IL-2 T cells from the control level in the wild type subject.

### BRIEF DESCRIPTION OF DRAWINGS

Figure 1 shows a comparison of the DQ8 and DR4 T cell responses of T1D patients treated with varying doses of methyldopa.
Figure 2 shows the glucose control achieved in individual human T1D patients treated with methyldopa over 3 months.
Figure 3 shows the beta-cell function in individual human T1D patients treated with methyldopa over 3 months.
Figure 4 shows the inhibition of DQ8 presentation to T cells in transgenic mice following administration of immediate release or extended release methyldopa formulations.

### DESCRIPTION OF EMBODIMENTS

The present disclosure is drawn to methods of preventing or treating autoimmune diabetes by reducing the binding of MHC class II molecules to antigenic peptides or fragments of antigenic peptides of the autoimmune disease by the administration of methyldopa to an individual having or suspected of having autoimmune diabetes (T1D).

The term "insulin peptide" is used to denote a peptide fragment of an insulin protein. Although the fragment is typically a subset of the amino acid sequence of the insulin protein, an insulin peptide may contain the entire amino acid sequence of a naturally-occurring insulin protein.

For the purposes of this disclosure, the term "methyldopa" includes methyldopa or any pharmaceutically acceptable salts thereof.

An "immediate release formulation" refers to a formulation that releases greater than or equal to about 80% of the pharmaceutical agent in less than or equal to about 1 hour.

"Extended release" is defined herein as delayed bioavailability of a pharmaceutical agent in a continuous manner over a prolonged period of time. By "prolonged period of time" it is meant a continuous period of time of greater than about 1 hour, preferably, greater than about 4 hours, more preferably, greater than about 8 hours, more preferably greater than about 12 hours, more preferably still, greater than about 16 hours up to more than about 24 hours. The continuous period of time may be between about 12 hours and about 24 hours.

As used herein, "rate of release" or "release rate" of a drug refers to the quantity of drug released from a dosage form per unit time, e.g., milligrams of drug released per hour (mg/hr) or a percentage of a total drug dose released per hour. Drug release rates for dosage forms are typically measured as an *in vitro* rate of drug release, i.e., a quantity of drug released from the dosage form per unit time measured under appropriate conditions and in a suitable fluid. The time at which a specified percentage of the drug within a dosage form has been released from the dosage form is referred to as the "Tₓ" value, where X is the percent of drug that has been released. The release rates referred to herein are determined by placing a dosage form to be tested in a medium in an appropriate dissolution bath. Aliquots of the medium, collected at pre-set intervals, are then injected into a chromatographic system fitted with an appropriate detector to quantify the amounts of drug released during the testing intervals.

"C" denotes the concentration of drug in blood plasma, or serum, of an individual, and is generally expressed as mass per unit volume, for example nanograms per milliliter. For convenience, this concentration may be referred to herein as "drug plasma concentration," "plasma drug concentration" or "plasma concentration" which is intended to be inclusive of a drug concentration measured in any appropriate body fluid or tissue. The plasma drug concentration at any time following drug administration is referenced as Ctime, as in C9 hr or C4 hr, etc.

The maximum plasma drug concentration during the dosing period is referenced as Cmax, while Cmin refers to the minimum blood plasma drug concentration at the end of a dosing interval; and Cave refers to an average concentration during the dosing interval.

The term "individual" or "subject" typically refers to humans, but also to mammals and other animals. "Tissue" means any sample taken from any individual, preferably a human. Tissues include blood, saliva, urine, biopsy samples, skin or buccal scrapings, and hair. Similarly, the term "patient" refers to both human and veterinary subjects.

Persons of skill in the art will appreciate that blood plasma drug concentrations obtained in individual subjects will vary due to inter-patient variability in the many parameters affecting drug absorption, distribution, metabolism and excretion. For this reason, unless otherwise indicated, when a drug plasma concentration is listed, the value listed is the calculated mean value based on values obtained from a group of subjects tested.

The term "bioavailability" refers to the extent to which, and sometimes rate at which, the active moiety (drug or metabolite) enters systemic circulation, thereby gaining access to the site of action.

"AUC" is the area under the plasma concentration-time curve and is considered to be the most reliable measure of bioavailability. It is directly proportional to the total amount of unchanged drug that reaches the systemic circulation.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

"Pharmaceutically-acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, or alkali or organic salts of acidic residues such as carboxylic acids. Pharmaceutically-acceptable salts include the conventional non-toxic salts or the quaternary ammonium salts of the parent compound formed, for example, from non-toxic inorganic or organic acids. Such conventional nontoxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. Pharmaceutically acceptable salts are those forms of compounds, suitable for use in contact with the tissues of human beings and animals without excessive toxicity, irritation, allergic response, or other problem or complication, commensurate with a reasonable benefit/risk ratio. Pharmaceutically acceptable salts may be chosen to complex with the methyldopa and delay the absorption of the methyldopa into the individual to whom the methyldopa salt is administered.

The term "therapeutically-effective amount" of methyldopa means an amount effective to modulate the formation or progression of autoimmune diabetes in an individual.

Methyldopa is α-Methyl-3,4-dihydroxyphenylalanine (available commercially under the tradenames ALDOMET^{™}, ALDORIL^{™}, DOPAMET^{™}, DOPEGYT^{™}) having the chemical structure:

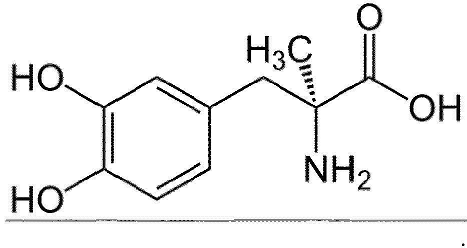

Methyldopa is an alpha-adrenergic agonist (selective for α2-adrenergic receptors) that was developed as a psychoactive drug and has been used extensively as a sympatholytic or antihypertensive.

It will be appreciated from the chemical structure above that methyldopa has a chiral center that may exist in, and may be isolated in, optically active and racemic forms. It is to be understood that the formulations of this disclosure encompass any racemic, optically-active, regioisomeric or stereoisomeric form, in isolation, or mixtures thereof, which possess the therapeutically useful properties related to the prevention and treatment of T1D described herein. It is well known in the art how to prepare optically active forms (for example, by resolution of the racemic form by recrystallization techniques, by synthesis from optically-active starting materials, by chiral synthesis, or by chromatographic separation using a chiral stationary phase). The scope of this disclosure encompasses not only the various isomers, which may exist but also the various mixtures of isomers, which may be formed. The resolution of methyldopa isomers, may be carried out by known procedures, e.g., as described in the four volume compendium Optical Resolution Procedures for Chemical Compounds: Optical Resolution Information Center, Manhattan College, Riverdale, N.Y., and in Enantiomers, Racemates and Resolutions, Jean Jacques, Andre Collet and Samuel H. Wilen; John Wiley & Sons, Inc., New York, 1981.

As noted above, methyldopa may be purchased commercially. Methyldopa may also be prepared in ways well known to one skilled in the art of organic synthesis.

This disclosure provides methods of preventing or treating autoimmune diabetes by reducing the binding of MHC class II molecules to antigenic peptides or fragments of antigenic peptides of the autoimmune disease by the administration of methyldopa to an individual having or suspected of having autoimmune diabetes (T1D).

In these methods of preventing or treating T1D, methyldopa or a pharmaceutically acceptable salt thereof, is administered to an individual suspected of having or developing T1D. Preferably, the administration to individual having T1D commences within 5 years of the initial diagnosis of T1D in the individual, or more preferably, within 1 year of the initial diagnosis of T1D in the individual, or more preferably, within 6 months of the initial diagnosis of T1D in the individual, or more preferably, within 1 month of the initial diagnosis of T1D in the individual.

In these methods, the methyldopa may be administered in dosages ranging between 100 mg and 5000 mg per day. Typically, the methyldopa is administered in dosages ranging between 250 mg and 3000 mg per day. In most instances, the methyldopa may be initially administered at a dosage of 250 mg to 500 mg once, twice, or 3 times daily. Methyldopa is currently available for administration has an immediate-release tablet, administered in multiple daily doses. Unfortunately, for long term administration, such as for long-term use in delaying the onset of T1D or in treating a patient diagnosed with T1D, compliance with a 2- or 3-times per day dosage regimen is low. This may be in part due to the psychological or neurological side effects exhibited by the immediate release product, especially when taken in high single dose(s). Therefore, in exemplary embodiments, methyldopa is administered in a single daily dosage of an extended release (XR) pharmaceutical formulation.

This disclosure provides extended-release pharmaceutical formulations of methyldopa. These formulations may be characterized by a maximum steady state plasma concentration (Cmax) of methyldopa which is higher than the minimal therapeutically effective concentration, and is in the range of 50% to 125% of the maximum plasma concentration produced by the same amount of methyldopa administered as an immediate release formulation twice daily. These extended release formulations may provide for a relative Cmax in the range of 80% to 125%, as compared to the same amount of methyldopa administered as an immediate release formulation twice daily. The Cmax of these extended release methyldopa formulations may be lower than the maximum plasma concentration produced by the same amount of methyldopa administered as an immediate release formulation twice daily. The Cmin of the methyldopa formulations may be about equal to, or higher than, a Cmin of an equivalent amount of immediate release methyldopa formulation given twice.

Compared to the immediate release methyldopa formulation, the extended release methyldopa formulations attenuate the Cmax of methyldopa while extending the coverage of plasma concentrations above the minimum plasma concentration required for therapeutic efficacy. These extended release formulations provide for a relative steady state AUC in the range of 80% to 125%, while minimizing the degree of fluctuation, which is preferably in the range of 25% to 90%, as compared to an equivalent amount of immediate release methyldopa formulation given in divided doses twice daily.

The relative amount of methyldopa in these extended release formulations varies from about 0.5 mg to about 5000 mg. In other words, methyldopa or its salt is present in the composition in an amount of from about 0.5% to about 85% by weight, and preferably of from about 2% to about 70% by weight. The term "about" is recited here and throughout the specification to account for variations, which can arise from inaccuracies in measurement inherent and understood by those of ordinary skill in the chemical and pharmaceutical arts.

The extended release component of these formulations may release methyldopa in a continuous manner and is adjusted in such a way that 80% of the active ingredient is released *in vitro* in the predetermined period of time. By way of non-limiting example, the period of time may be not more than 24 hours, not more than 16 hours, not more than 12 hours, not more than 8 hours, or not more than 4 hours, depending on desired attributes of the final product.

The extended release methyldopa formulations comprise an oil-in-water emulsion. The oil is olive oil Additionally, the oil comprises lecithin. Such oil-in-water emulsion extended-release methyldopa formulations may be made by first acidifying the methyldopa, and then introducing the acidified methyldopa into an oil-in-water emulsion. The resulting oil-in-water emulsion may be administered orally as a liquid, or it may be encapsulated, such as within a liquid capsule (LiquiCaps), for oral administration.

These extended release methyldopa formulations are suitable for once-daily administration, and result in better patient compliance for long-term administration as well as diminished levels or severity of side effects.

In addition to the specific oil-in-water emulsion extended-release methyldopa formulations, also provided herein are pharmaceutical compositions containing methyldopa and a pharmaceutically-acceptable carrier, which are media generally accepted in the art for the delivery of biologically active agents to animals, in particular, mammals. Pharmaceutically-acceptable carriers are formulated according to a number of factors well within the purview of those of ordinary skill in the art to determine and accommodate. These include, without limitation: the type and nature of the active agent being formulated; the subject to which the agent-containing composition is to be administered; the intended route of administration of the composition; and, the therapeutic indication being targeted. Pharmaceutically-acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, well known to those of ordinary skill in the art. Descriptions of suitable pharmaceutically-acceptable carriers, and factors involved in their selection, are found in a variety of readily available sources, such as Remington's Pharmaceutical Sciences, 17th ed., Mack Publishing Company, Easton, Pa., 1985.

Further disclosed are methods of preventing or treating an individual afflicted with T1D, which includes administering to the individual a pharmaceutical composition provided herein. Such compositions generally comprise a therapeutically effective amount of methyldopa, which is an amount effective to reduce the incidence or onset, ameliorate, lessen, or inhibit T1D in the individual. Such amounts typically comprise from about 100 to about 3000 mg of methyldopa. Therapeutically effective amounts of methyldopa can be administered according to the methods of this disclosure by any dosing regimen satisfactory to the prescribing healthcare practitioner and/or the individual being treated. In example embodiments, the methyldopa is administered in an extended release pharmaceutical formulation on a once or twice daily basis.

Formulations of methyldopa mentioned in the following paragraphs other than the extended release formulation according to the claims are not according to the invention.

Administration may be, for example, administered orally in solid dosage forms, such as capsules, tablets and powders; or in liquid forms such as elixirs, syrups, and/or suspensions. Gelatin capsules can be used to contain the active ingredient and a suitable carrier such as, but not limited to, lactose, starch, magnesium stearate, stearic acid, or cellulose derivatives. Similar diluents can be used to make compressed tablets. Both tablets and capsules can be manufactured as extended release products to provide for continuous release of medication over an extended period of time that is longer than the release time of traditional 'immediate release' oral dosage forms. Compressed tablets can be sugar-coated or film-coated to mask any unpleasant taste, or used to protect the active ingredients from the atmosphere, or to allow selective disintegration of the tablet in the gastrointestinal tract.

Alternatively, compositions can be administered by various parenteral means. Pharmaceutical compositions suitable for parenteral administration include various aqueous media such as aqueous dextrose and saline solutions; glycol solutions are also useful carriers, and preferably contain a water soluble salt of the active ingredient, suitable stabilizing agents, and if necessary, buffering agents. Antioxidizing agents, such as sodium bisulfite, sodium sulfite, or ascorbic acid, either alone or in combination, are suitable stabilizing agents; also used are citric acid and its salts, and EDTA. In addition, parenteral solutions can contain preservatives such as benzalkonium chloride, and methyl- or propyl-paraben.

A preferred formulation is a mono-phasic pharmaceutical composition suitable for oral administration for the prevention, treatment or prophylaxis of autoimmune diabetes, consisting essentially of a therapeutically-effective amount of methyldopa, and a pharmaceutically acceptable carrier, in an extended release format.

Examples of suitable aqueous and nonaqueous carriers which may be employed in these pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monosterate and gelatin.

In order to prolong the effect of the methyldopa, it may be desirable to slow the absorption of methyldopa from subcutaneous or intramuscular injection. This may be accomplished by the use of a liquid suspension of crystalline or amorphous material having poor water solubility. The rate of absorption of the methyldopa then depends upon its rate of dissolution, which in turn may depend upon crystal size and crystalline form. Alternatively, delayed absorption of a parenterally-administered methyldopa is accomplished by dissolving or suspending the methyldopa in an oil vehicle, which oil may comprise a vegetable oil, for example olive oil.

Injectable depot forms are made by forming microencapsule matrices of methyldopa in biodegradable polymers such as polylactide-polyglycolide. Depending on the ratio of methyldopa to polymer, and the nature of the particular polymer employed, the rate of methyldopa release can be controlled. Examples of other biodegradable polymers include poly(orthoesters) and poly(anhydrides). Depot injectable formulations are also prepared by entrapping the methyldopa in liposomes or microemulsions which are compatible with body tissue.

Formulations of this disclosure suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of methyldopa as an active ingredient.

In solid dosage forms for oral administration (capsules, tablets, pills, dragees, powders, granules and the like), the methyldopa is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a machine a mixture of the powdered compound moistened with an inert liquid diluent.

The tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide extended or controlled release of the active ingredient therein using, for example, hydroxypropylmethyl cellulose in varying proportions to provide the desired release profile, other polymer matrices, liposomes and/or microspheres. These compositions may also optionally contain opacifying agents and may be of a composition that they release the active ingredient only, or preferentially, in a certain portion of the gastrointestinal tract, optionally, in a delayed manner. Examples of embedding compositions which can be used include polymeric substances and waxes. The active ingredient can also be in microencapsulated form.

The tablets or pills may be coated or otherwise compounded to provide a dosage form affording the advantage of extended-release. For example, the tablet or pill can comprise an inner dosage and an outer dosage component, the latter being in the form of an envelope over the former. A variety of materials can be used for such enteric layers or coatings, such materials including a number of polymeric acids and mixtures of polymeric acids with such materials as shellac, cetyl alcohol, and cellulose acetate.

Release modifying polymers may be used to form extended release formulations containing methyldopa. The release modifying polymers may be either water-soluble polymers, or water insoluble polymers. Examples of water-soluble polymers include polyvinylpyrrolidone, hydroxy propylcellulose, hydroxypropyl methylcellulose, vinyl acetate copolymers, polyethylene oxide, polysaccharides (such as alginate, xanthan gum, etc.), methylcellulose and mixtures thereof. Examples of water-insoluble polymers include acrylates such as methacrylates, acrylic acid copolymers; cellulose derivatives such as ethylcellulose or cellulose acetate; polyethylene, and high molecular weight polyvinyl alcohols.

Liquid dosage forms for oral administration of methyldopa include pharmaceutically-acceptable emulsions, microemulsions, solutions, suspensions, syrups and elixirs. In addition to methyldopa, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of this disclosure for rectal or vaginal administration may be prepared as a suppository, which may be prepared by mixing methyldopa with one or more suitable nonirritating excipients or carriers comprising, for example, cocoa butter, polyethylene glycol, a suppository wax or salicylate, and which is solid at room temperature, but liquid at body temperature and, therefore, will melt in the rectum or vaginal cavity and release the active compound. Formulations which are suitable for vaginal administration also include pessaries, tampons, creams, gels, pastes, foams or spray formulations containing such carriers as are known in the art to be appropriate.

Dosage forms for the topical or transdermal administration of methyldopa include powders, sprays, ointments, pastes, creams, lotions, gels, solutions, patches, drops and inhalants. The ointments, pastes, creams and gels may contain, in addition to methyldopa, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Transdermal patches have the added advantage of providing controlled delivery of methyldopa to the body of an individual. Such dosage forms can be made by dissolving, dispersing or otherwise incorporating methyldopa in a proper medium, such as an elastomeric matrix material. Absorption enhancers can also be used to increase the flux of the compound across the skin. The rate of such flux can be controlled by either providing a rate-controlling membrane or dispersing the compound in a polymer matrix or gel.

The methyldopa formulations of this disclosure may be presented in unit-dose or multi-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

Suitable antioxidants may be selected from amongst one or more pharmaceutically acceptable antioxidants known in the art. Examples of pharmaceutically acceptable antioxidants include butylated hydroxyanisole (BHA), sodium ascorbate, butylated hydroxytoluene (BHT), sodium sulfite, citric acid, malic acid and ascorbic acid. The antioxidants may be present in these dosage formulations at a concentration between about 0.001% to about 5%, by weight, of the dosage formulation.

Suitable chelating agents may be selected from amongst one or more chelating agents known in the art. Examples of suitable chelating agents include disodium edetate (EDTA), edetic acid, citric acid and combinations thereof. The chelating agents may be present in a concentration between about 0.001% and about 5%, by weight, of the dosage formulation.

These formulations may include one or more diluents such as lactose, sugar, cornstarch, modified cornstarch, mannitol, sorbitol, and/or cellulose derivatives such as wood cellulose and microcrystalline cellulose, typically in an amount within the range of from about 20% to about 80%, by weight.

These formulations may include one or more binders in an amount of up to about 60% w/w. Examples of suitable binders include methyl cellulose, hydroxypropyl cellulose, hydroxypropylmethyl cellulose, polyvinyl pyrrolidone, eudragits, ethyl cellulose, gelatin, gum arabic, polyvinyl alcohol, pullulan, carbomer, pregelatinized starch, agar, tragacanth, sodium alginate, microcrystalline cellulose and the like.

Examples of suitable disintegrants include sodium starch glycolate, croscarmellose sodium, crospovidone, low substituted hydroxypropyl cellulose, and the like. The concentration may vary from 0.1% to 15%, by weight, of the dosage form.

Examples of lubricants/glidants include colloidal silicon dioxide, stearic acid, magnesium stearate, calcium stearate, talc, hydrogenated castor oil, sucrose esters of fatty acid, microcrystalline wax, yellow beeswax, white beeswax, and the like. The concentration may vary from 0.1% to 15%, by weight, of the dosage form.

### EXAMPLES

### Example 1 Human T1D treatment study

Human leukocyte antigen (HLA) alleles confer significant genetic risk for type 1 diabetes (T1D) with recent studies implicating DQ8 in its pathogenesis, and DQ8 antigen presentation can be inhibited with methyldopa in animal models. In this pilot study, the inventors evaluated methyldopa treatment in 10 DQ8 positive human subjects with T1D, ages 18-46 years (mean 27) with less than 2 years of diabetes duration (mean 3 months). This was an open label phase 1b dose escalation study. All subjects tolerated low (500mg BID) and moderate (500mg TID) dosages of methyldopa, while 9/10 tolerated the high dose (2-3g/day).

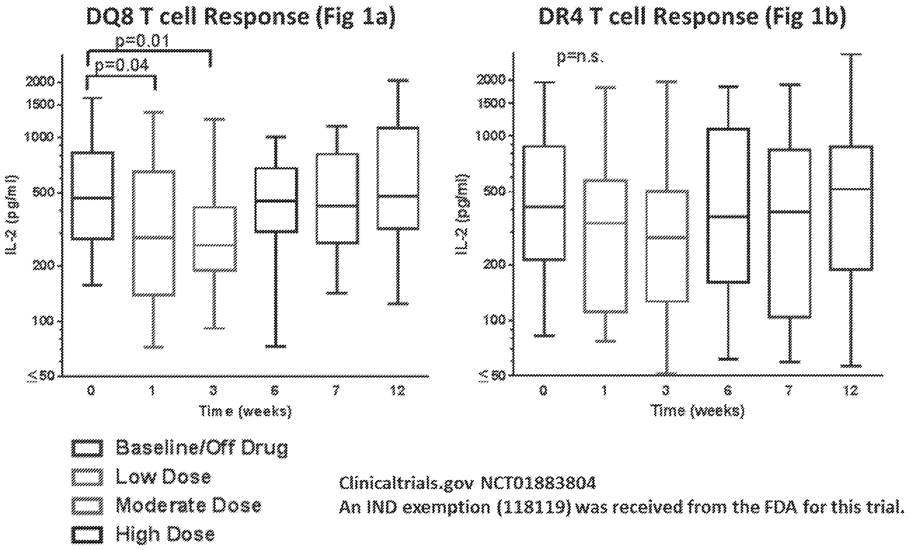

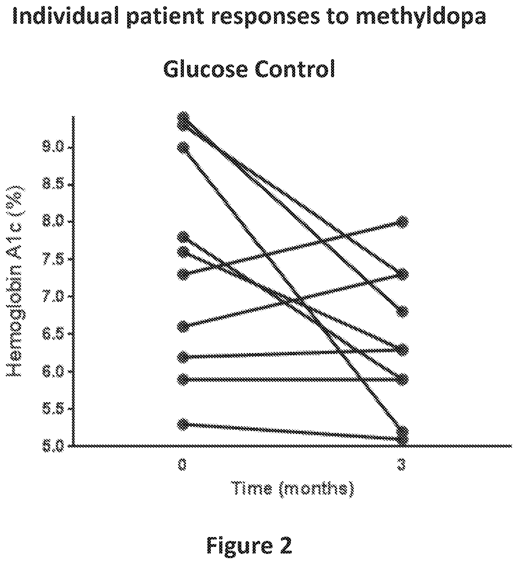

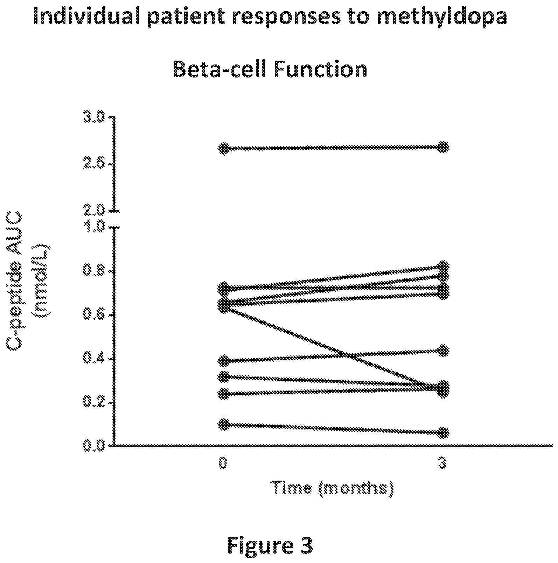

There was a dose dependent reduction in DQ8-stimulated IL-2 T cell response at 1 (-32%) and 3 weeks (-39%), which returned to normal 6 weeks after stopping therapy (Figure 1a). This response was specific for the DQ8-stimulated T cell response because DR4 T cell responses were minimally effected (Figure 1b). The treated T1D patients had good glycemic control (Figure 2). Additionally, the 2 hour AUC for C-peptide following a mixed meal tolerance test at 12 weeks was similar to baseline levels (Figure 3). No serious adverse events (hypotension, DKA, or hypoglycemia) were reported throughout the study. This example demonstrates that methyldopa inhibits DQ8 antigen presentation in TID.

### Example 2 Preparation of an extended release formulation of methyldopa.

An extended release formulation of methyldopa suitable for oral administration was formed by dissolving methyldopa in an acid, and emulsifying the acidic solution in an oil phase consisting of olive oil and lecithin (3:1 w/w). This formulation effectively slows the release of the methyldopa from the formulation compared to the release profile of methyldopa from immediate release formulations commercially available and known in the art. Without intending to be bound by theory, the extended release profile of the methyldopa from this oil emulsion formulation may result from slow or delayed digestion of the formulation, and/or ion-pairing between the lecithin and the methyldopa, and/or encapsulation of the methyldopa in micelles/liposomes which slows release in the GI tract of the individual.

### Example 3 Extended release study of methyldopa in mice

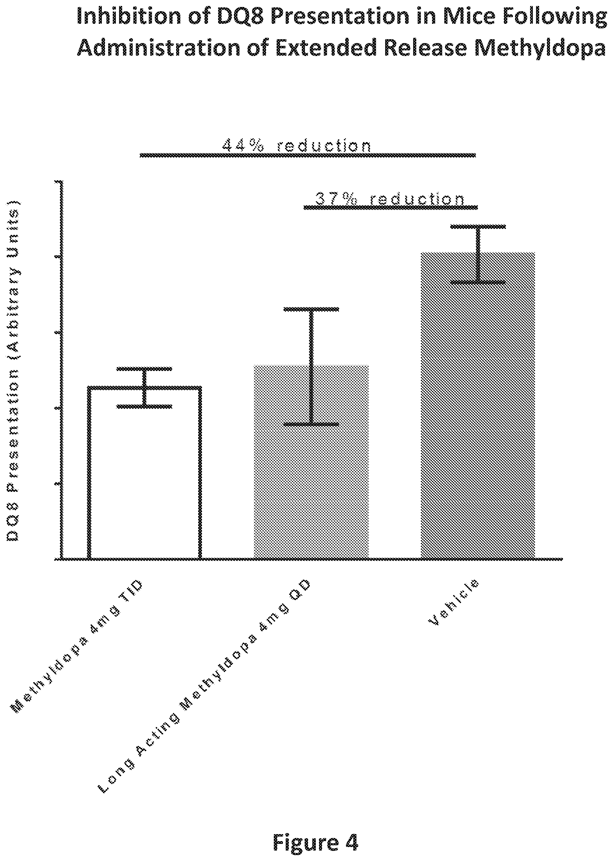

Methyldopa, and an extended release formulation of methyldopa, and a vehicle control was administered via oral gavage to HLA-DQ8 transgenic mice (n=3 per group) over 24 hours. Rapid acting methyldopa was administered in 4mg doses three times daily, while 4mg of the extended release version was administered once, and a vehicle control for the extended release version was administered once. The extended release version of methyldopa is complexed with olive oil and lecithin as described previously. A bioassay to determine the amount of DQ8 presentation to a responding CD4 T cell hybridoma was determined and compared between groups. There is a similar reduction in the function of HLA-DQ8 with both the rapid and the extended release version of methyldopa (Figure 4).

## Claims

1. An extended release formulation of methyldopa for use in delaying the onset or the treatment of an autoimmune disorder including T1D and/or Celiac disease in an individual, wherein the formulation comprises an oil-in-water emulsion comprising an oil phase consisting of olive oil and lecithin and wherein the methyldopa present in the formulation is absorbed into the body of the individual over a period of time between 14 hours and 24 hours following oral administration of the formulation to the individual.

2. The extended release formulation of claim 1 for use according to claim 1, wherein the olive oil and lecithin are present in a ratio of about 3:1 w/w.

3. The formulation of claims 1 or 2 for use of claim 1 or 2, wherein the individual is between 1 year and 30 years of age.

4. The formulation of any one of claims 1 to 3, for use of any one of claims 1-3, wherein the individual is administered a dosage of methyldopa between 50mg and 3000mg per day.

5. The formulation of claim 1, for use of claim 1, wherein the individual is administered a dosage of methyldopa between about 250 mg and about 1000 mg per day.

6. The formulation of any one of claims 1 to 3, for use of any one of claims 1 to 3, wherein the individual is administered a single daily dosage of an extended-release pharmaceutical formulation of methyldopa.

7. The formulation of claim 6, for use of claim 6, wherein the single daily dosage is administered at bedtime.

8. The formulation of claim 1, for use of claim 1, wherein the formulation is for administration in combination with an anti-diabetic compound selected from at least one of an alpha-glucosidase inhibitor, a biguanide, a Dpp-4 inhibitor, a meglitinide, a sulfonylurea, a thiazolidinedione or combinations thereof.

9. The formulation of claim 1, for use of claim 1, wherein the individual has been tested for the presence of antibodies to a MHC class II molecule, wherein the presence of antibodies that recognize the MHC class II molecules is indicative of the presence or likely development of an autoimmune disorder.

10. The formulation of claim 9, for use of claim 9, wherein the individual is found to have antibodies to a MHC class II molecule bound to an insulin protein or to a peptide fragment of an insulin peptide.

11. A pharmaceutical composition comprising methyldopa formulated in an oral extended release pharmaceutical formulation, wherein the formulation comprises an oil-in-water emulsion comprising an oil phase consisting of olive oil and lecithin and wherein the methyldopa component of the pharmaceutical formulation is absorbed from the formulation to the body of an individual over a period of between about 14 hours to about 24 hours, following oral administration of the formulation to the individual.

12. The pharmaceutical composition of claim 11, wherein the extended release pharmaceutical formulation comprises a pharmaceutically acceptable salt of methyldopa.

13. An extended release formulation of methyldopa for use according to claim 1 wherein the dosage of methyldopa administered to the individual is monitored and adjusted using a method comprising:
a. Receiving a blood sample from an individual having or suspected of developing an autoimmune disorder that has been administered methyldopa;
b. Determining the DQ8-stimulated response of IL-2 T cells in the blood sample;
c. Comparing the DQ8-stimulated response of IL-2 T cells in the blood sample to a control level of DQ8-stimulated response of IL-2 T cells in blood samples from at least one of a patient having the autoimmune disorder and a wild type subject known to be free of the autoimmune disorder; and
d. Increasing the dosage and/or the frequency of the methyldopa administered to the individual if the DQ8-stimulated response of IL-2 T cells in the blood sample from the individual is statistically similar to the DQ8-stimulated response of IL-2 T cells from the control level in the patient having the autoimmune disorder; or
e. Maintaining or decreasing the dosage and/or the frequency of the methyldopa administered to the individual if the DQ8-stimulated response of IL-2 T cells in the blood sample from the individual is statistically similar to the DQ8-stimulated response of IL-2 T cells from the control level in the wild type subject.

14. The formulation of claim 1 for use of claim 1, or the pharmaceutical composition of claim 11, wherein the extended release pharmaceutical formulation comprises a stereoisomeric form of methyldopa in isolation.

## Patentansprüche

1. Methyldopa-Formulierung mit verlängerter Freisetzung, zur Verwendung beim Verzögern des Ausbruchs oder beim Behandeln einer Autoimmunstörung einschließlich T1D und/oder Zöliakie bei einem Individuum, wobei die Formulierung eine Öl-in-Wasser-Emulsion umfasst, die eine aus Olivenöl und Lecithin bestehende Ölphase umfasst, und wobei das in der Formulierung vorhandene Methyldopa in den Körper des Individuums über einen Zeitraum von zwischen 14 Stunden und 24 Stunden im Anschluss an orale Verabreichung der Formulierung an das Individuum resorbiert wird.

2. Formulierung mit verlängerter Freisetzung nach Anspruch 1, zur Verwendung nach Anspruch 1, wobei das Olivenöl und das Lecithin in einem Verhältnis von ungefähr 3:1 Gew./Gew. vorliegen.

3. Formulierung nach den Ansprüchen 1 oder 2, zur Verwendung nach den Ansprüchen 1 oder 2, wobei das Individuum zwischen 1 Jahr und 30 Jahren alt ist.

4. Formulierung nach einem der Ansprüche 1 bis 3, zur Verwendung nach einem der Ansprüche 1-3, wobei dem Individuum eine Dosis Methyldopa von zwischen 50 mg und 3000 mg pro Tag verabreicht wird.

5. Formulierung nach Anspruch 1, zur Verwendung nach Anspruch 1, wobei dem Individuum eine Dosis Methyldopa von zwischen ungefähr 250 mg und ungefähr 1000 mg pro Tag verabreicht wird.

6. Formulierung nach einem der Ansprüche 1 bis 3, zur Verwendung nach einem der Ansprüche 1 bis 3, wobei dem Individuum eine tägliche Einzeldosis einer pharmazeutischen Methyldopa-Formulierung mit verlängerter Freisetzung verabreicht wird.

7. Formulierung nach Anspruch 6, zur Verwendung nach Anspruch 6, wobei die tägliche Einzeldosis zum Zeitpunkt des Schlafengehens verabreicht wird.

8. Formulierung nach Anspruch 1, zur Verwendung nach Anspruch 1, wobei die Formulierung zur Verabreichung in Kombination mit einer antidiabetischen Verbindung ist, ausgewählt aus mindestens einem von einem Alpha-Glukosidase-Hemmer, einem Biguanid, einem Dpp-4-Hemmer, einem Meglitinid, einem Sulfonylharnstoff, einem Thiazolidindion oder Kombinationen davon.

9. Formulierung nach Anspruch 1, zur Verwendung nach Anspruch 1, wobei das Individuum auf das Vorliegen von Antikörpern gegen ein MHC-Klasse-II-Molekül getestet worden ist, wobei das Vorliegen von Antikörpern, welche die MHC-Klasse-II-Moleküle erkennen, ein Indikator für das Vorliegen oder die wahrscheinliche Entwicklung einer Autoimmunstörung ist.

10. Formulierung nach Anspruch 9, zur Verwendung nach Anspruch 9, wobei beim Individuum festgestellt wird, dass es Antikörper gegen ein MHC-Klasse-II-Molekül, gebunden an ein Insulinprotein oder an ein Peptidfragment eines Insulinpeptids, aufweist.

11. Pharmazeutische Zusammensetzung, umfassend Methyldopa, formuliert in einer oralen pharmazeutischen Formulierung mit verlängerter Freisetzung, wobei die Formulierung eine Öl-in-Wasser-Emulsion umfasst, die eine aus Olivenöl und Lecithin bestehende Ölphase umfasst, und wobei die Methyldopa-Komponente der pharmazeutischen Formulierung aus der Formulierung in den Körper eines Individuums über einen Zeitraum von zwischen ungefähr 14 Stunden bis ungefähr 24 Stunden resorbiert wird, im Anschluss an orale Verabreichung der Formulierung an das Individuum.

12. Pharmazeutische Zusammensetzung nach Anspruch 11, wobei die pharmazeutische Formulierung mit verlängerter Freisetzung ein pharmazeutisch verträgliches Salz von Methyldopa umfasst.

13. Methyldopa-Formulierung mit verlängerter Freisetzung, zur Verwendung nach Anspruch 1, wobei die Methyldopa-Dosis, die dem Individuum verabreicht wird, mittels eines Verfahrens überwacht und eingestellt wird, das umfasst:
a. Erhalten einer Blutprobe von einem Individuum mit einer Autoimmunstörung oder bei Verdacht auf Entwicklung davon, dem Methyldopa verabreicht worden ist;
b. Bestimmen des DQ8-stimulierten Ansprechens von IL-2 T-Zellen in der Blutprobe;
c. Vergleichen des DQ8-stimulierten Ansprechens von IL-2 T-Zellen in der Blutprobe mit einem Kontrolllevel DQ8-stimulierten Ansprechens von IL-2 T-Zellen in Blutproben von mindestens einem von einem Patienten mit der Autoimmunstörung und einem Wildtyp-Individuum, das bekanntermaßen frei von der Autoimmunstörung ist; und
d. Erhöhen der Dosis und/oder der Häufigkeit des dem Individuum verabreichten Methyldopas, falls das DQ8-stimulierte Ansprechen von IL-2 T-Zellen in der Blutprobe vom Individuum statistisch dem DQ8-stimulierten Ansprechen von IL-2 T-Zellen aus dem Kontrolllevel beim Patienten mit der Autoimmunstörung ähnlich ist; oder
e. Beibehalten oder Verringern der Dosis und/oder der Häufigkeit des dem Individuum verabreichten Methyldopas, falls das DQ8-stimulierte Ansprechen von IL-2 T-Zellen in der Blutprobe vom Individuum statistisch dem DQ8-stimulierten Ansprechen von IL-2 T-Zellen aus dem Kontrolllevel beim Wildtyp-Individuum ähnlich ist.

14. Formulierung nach Anspruch 1, zur Verwendung nach Anspruch 1, oder pharmazeutische Zusammensetzung nach Anspruch 11, wobei die pharmazeutische Formulierung mit verlängerter Freisetzung eine stereoisomere Form von Methyldopa in Isolation umfasst.

## Revendications

1. Formulation à libération prolongée de méthyldopa pour utilisation dans le retardement de l'apparition ou le traitement d'un trouble auto-immun incluant T1D et/ou une maladie cœliaque chez un individu, où la formulation comprend une émulsion d'huile dans l'eau comprenant une phase huileuse constituée d'huile d'olive et de lécithine et où le méthyldopa présent dans la formulation est absorbé dans le corps de l'individu sur une période de temps d'entre 14 heures et 24 heures à la suite d'une administration orale de la formulation à l'individu.

2. Formulation à libération prolongée selon la revendication 1 pour utilisation selon la revendication 1, dans laquelle l'huile d'olive et la lécithine sont présentes dans un rapport d'environ 3:1 p/p.

3. Formulation selon les revendications 1 ou 2 pour utilisation selon les revendications 1 ou 2, où l'individu est âgé d'entre 1 an et 30 ans.

4. Formulation selon l'une quelconque des revendications 1 à 3, pour utilisation selon l'une quelconque des revendications 1 à 3, où une dose de méthyldopa d'entre 50 mg et 3000 mg par jour est administrée à l'individu.

5. Formulation selon la revendication 1, pour utilisation selon la revendication 1, où une dose de méthyldopa d'entre environ 250 mg et environ 1000 mg par jour est administrée à l'individu.

6. Formulation selon l'une quelconque des revendications 1 à 3, pour utilisation selon l'une quelconque des revendications 1 à 3, où une seule dose quotidienne d'une formulation pharmaceutique à libération prolongée de méthyldopa est administrée à l'individu.

7. Formulation selon la revendication 6, pour utilisation selon la revendication 6, où la seule dose quotidienne est administrée au coucher.

8. Formulation selon la revendication 1, pour utilisation selon la revendication 1, où la formulation est pour une administration en combinaison avec un composé antidiabétique choisi parmi au moins un d'un inhibiteur d'alpha-glucosidase, d'un biguanide, d'un inhibiteur de Dpp-4, d'un méglitinide, d'une sulfonylurée, d'une thiazolidinedione ou des combinaisons de ceux-ci.

9. Formulation selon la revendication 1, pour utilisation selon la revendication 1, où l'individu a été testé sur la présence d'anticorps contre une molécule MHC de classe II, où la présence d'anticorps qui reconnaissent les molécules MHC de classe II est une indication de la présence ou du développement probable d'un trouble auto-immun.

10. Formulation selon la revendication 9, pour utilisation selon la revendication 9, où il est trouvé que l'individu possède des anticorps contre une molécule MHC de classe II liée à une protéine d'insuline ou un fragment peptidique d'un peptide d'insuline.

11. Composition pharmaceutique comprenant du méthyldopa formulé en une formulation pharmaceutique à libération prolongée orale, où la formulation comprend une émulsion d'huile dans l'eau comprenant une phase huileuse constituée d'huile d'olive et de lécithine et où le composant de méthyldopa de la formulation pharmaceutique est absorbé depuis la formulation vers le corps d'un individu sur une période de temps d'entre environ 14 heures et environ 24 heures, à la suite d'une administration orale de la formulation à l'individu.

12. Composition pharmaceutique selon la revendication 11, où la formulation pharmaceutique à libération prolongée comprend un sel pharmaceutiquement acceptable de méthyldopa.

13. Formulation à libération prolongée de méthyldopa pour utilisation selon la revendication 1, où la dose de méthyldopa administrée à l'individu est surveillée et ajustée en utilisant un procédé comprenant:
a. la réception d'un échantillon de sang provenant d'un individu ayant ou suspecté de développer un trouble auto-immun auquel il a été administré du méthyldopa;
b. la détermination de la réponse stimulée par DQ8 de cellules T IL-2 dans l'échantillon de sang;
c. la comparaison de la réponse stimulée par DQ8 de cellules T IL-2 dans l'échantillon de sang à un niveau témoin de réponse stimulée par DQ8 de cellules T IL-2 dans des échantillons de sang provenant d'au moins un d'un patient ayant le trouble auto-immun et d'un sujet de type sauvage connu pour être sans trouble auto-immun; et
d. l'augmentation de la dose et/ou de la fréquence du méthyldopa administré à l'individu si la réponse stimulée par DQ8 de cellules T IL-2 dans l'échantillon de sang provenant de l'individu est statistiquement similaire à la réponse stimulée par DQ8 de cellules T IL-2 provenant du niveau témoin chez le patient ayant le trouble auto-immun; ou
e. le maintien ou la diminution de la dose et/ou de la fréquence du méthyldopa administré à l'individu si la réponse stimulée par DQ8 de cellules T IL-2 dans l'échantillon de sang provenant de l'individu est statistiquement similaire à la réponse stimulée par DQ8 de cellules T IL-2 provenant du niveau témoin chez le sujet de type sauvage.

14. Formulation selon la revendication 1 pour utilisation selon la revendication 1 ou composition pharmaceutique selon la revendication 11, où la formulation pharmaceutique à libération prolongée comprend une forme stéréoisomère de méthyldopa lorsqu'il est isolé.
